# EUROPÄISCHE PATENTSCHRIFT

(11) **EP 0 961 768 B1**
(45) Veröffentlichungstag und Bekanntmachung des Hinweises auf die Patenterteilung: **19.09.2001**
(21) Anmeldenummer: 98905375.6
(22) Anmeldetag: 30.01.1998
(51) Int. Cl.: C07C 253/34

(54) **VERFAHREN ZUR ABTRENNUNG VON 6-AMINOCAPRONSÄURENITRIL AUS MISCHUNGEN, DIE 6-AMINOCAPRONSÄURENITRIL UND EIN IMIN ENTHALTEN**
METHOD FOR SEPARATING 6-AMINOCAPROIC ACID NITRILE FROM MIXTURES CONTAINING 6-AMINOCAPROIC ACID NITRILE AND AN IMINE
PROCEDE DE SEPARATION DE NITRILE D'ACIDE 6-AMINOCAPROIQUE COMPRIS DANS DES MELANGES CONTENANT DU NITRILE D'ACIDE 6-AMINOCAPROIQUE ET UNE IMINE

(30) Priorität: 07.02.1997 DE 19704613
(43) Veröffentlichungstag der Anmeldung: 08.12.1999
(73) Patentinhaber: BASF AKTIENGESELLSCHAFT, 67056 Ludwigshafen (DE)
(72) Erfinder: REHFINGER, Alwin, D-67112 Mutterstadt (DE); LUYKEN, Hermann, D-67069 Ludwigshafen (DE)
(86) Internationale Anmeldenummer: EP9800505
(87) Internationale Veröffentlichungsnummer: WO9834912

(56) Entgegenhaltungen:
- US-A- 5 133 838
- US-A- 5 153 351
- US-A- 5 162 567
- US-A- 5 192 399

## Beschreibung

Die vorliegende Erfindung betrifft ein Verfahren zur destillativen Abtrennung von 6-Aminocapronsäurenitril aus Mischungen (I), die 6-Aminocapronsäurenitril und ein Imin (II) enthalten.

Die partielle Hydrierung von Adipodinitril zu 6-Amincapronitril in Gegenwart eines Katalysators auf der Basis eines Metalls wie Nickel, Cobalt, Eisen, Rhodium oder Ruthenium ist allgemein bekannt, beispielsweise aus EP-A-161 419, EP-A-77 911, US-A-4,389,348, US-A-4,601,859, WO 93/1207, DE-A 42 35 466, DE-A 19 500 222 und der Deutschen Anmeldung 19 548 289.1.

Als Nebenprodukte entstehen unter anderem Imine, insbesondere Tetrahydroazepin der Formel 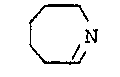 6-Aminocapronsäurenitril findet vor allem Verwendung für die Faserherstellung über Caprolactam als Zwischenstufe oder durch direkte Polymerisation zu Nylon 6. Hierzu muß das 6-Aminocapronsäurenitril eine hohe Reinheit aufweisen, wobei bekanntermaßen die Abtrennung von Tetrahydroazepin Probleme bereitet.

Aus US-A-5,162,567 ist bekannt, eine Mischung enthaltend 6-Aminocapronsäurenitril und Tetrahydroazepin mit einer organischen Carbonylverbindung, beispielsweise einem Keton oder einem Aldehyd, bei hoher Temperatur umzusetzen und anschließend 6-Aminocapronsäurenitril von der Mischung abzutrennen. Aus US-A-5,153,351 ist bekannt, eine Mischung enthaltend 6-Aminocapronsäurenitril und Tetrahydroazepin mit einer organischen C-H-aktiven Methylenverbindung, beispielsweise Malonitril, Cyclopentadien, Nitromethan oder Nitroethan, umzusetzen und anschließend 6-Aminocapronsäurenitril von der Mischung abzutrennen.

Der Nachteil bei diesen Verfahren besteht darin, daß die Zugabe einer weiteren organischen Verbindung zu der Mischung den Aufwand bei der Reindarstellung von 6-Aminocapronsäurenitril erhöht.

Gemäß US-A-5,133,838 wird eine Mischung enthaltend 6-Aminocapronsäurenitril und Tetrahydroazepin mit einem anorganischen Hydrid wie Lithiumborhydrid umgesetzt. Nachteiligerweise muß bei diesem Verfahren das Hydrid in mehrfachem Überschuß der stöchiometrisch benötigten Menge eingesetzt werden. Zudem muß bei der anschließenden Destillation Sorge getragen werden, daß nicht das Wertprodukt 6-Aminocapronsäurenitril hydriert wird.

Die EP-A-497 333 beschreibt ein Verfahren, gemäß dem eine Mischung enthaltend 6-Aminocapronsäurenitril und Tetrahydroazepin mit einer alkalischen Verbindung umgesetzt wird. Nachteiligerweise muß die alkalische Verbindung im Überschuß der stöchiometrisch benötigten Menge eingesetzt und aus der erhaltenen Reaktionsmischung das 6-Aminocapronsäurenitril bei stark vermindertem Druck abdestilliert werden.

Aus EP-A-628 025 ist bekannt, Mischungen enthaltend 6-Aminocapronsäurenitril und Tetrahydroazepin vor der Destillation auf 235°C zu erhitzen, um Tetrahydroazepin in Verbindungen umzuwandeln, die von 6-Aminocapronsäurenitril abdestillierbar sind.

Aufgabe der vorliegenden Erfindung war es daher, ein Verfahren bereitzustellen, das die Abtrennung von 6-Aminocapronsäurenitril aus einer Mischung, die im wesentlichen 6-Aminocapronsäurenitril und Tetrahydroazepin enthält, auf technisch einfache und wirtschaftliche Weise unter Überwindung der genannten Nachteile bereitzustellen.

Demgemäß wurde ein Verfahren zur destillativen Abtrennung von 6-Aminocapronsäurenitril aus Mischungen (I), die 6-Aminocapronsäurenitril und ein Imin (II) enthalten, dadurch gekennzeichnet, daß man die Destillation in einer Destillationskolonne durchführt und daß dabei auf mindestens einer Ebene der Destillationskolonne das Destillationsgemisch eine durchschnittliche mittlere Verweilzeit von mindestens 5 Minuten aufweist, gefunden.

Mischungen (I) können in an sich bekannter Weise erhalten werden durch partielle Hydrierung von Adipodinitril , beispielsweise nach einem Verfahren gemäß EP-A-161 419, EP-A-77 911, US-A-4,389,348, US-A-4,601,859, WO 93/1207, DE-A 42 35 466, DE-A 19 500 222 und der Deutschen Anmeldung 19 548 289.1, indem man im allgemeinen die Hydrierung in Gegenwart von Nickel-, Cobalt-, Eisen-, Rhodium- oder Ruthenium-haltigen Katalysatoren durchführt. Dabei können die Katalysatoren als Trägerkatalysatoren oder als Vollkatalysatoren verwendet werden. Als Katalysatorträger kommen beispielsweise Aluminiumoxid, Siliciumdioxid, Titandioxid, Magnesiumoxid, Aktivkohlen und Spinelle in Frage. Als Vollkatalysatoren kommen beispielsweise Raney-Nickel und Raney-Cobalt in Betracht.

Bei der Hydrierung erhält man ein Gemisch, das 6-Aminocapronsäurenitril, Hexamethylendiamin, ein Imin (II) und gegebenenfalls Adipodinitril enthält.

Aus diesem Gemisch kann eine Mischung (I), die im wesentlichen 6-Aminocapronsäurenitril und ein Imin (II) enthält, beispielsweise durch Destillation erhalten werden.

Als Imin (II) kommen aromatische, vorzugsweise aliphatische, wie acyclische oder insbesondere cyclische Imine, sowie deren Gemische, besonders bevorzugt Tetrahydroazepin in Betracht.

Für die Destillation kommen hierfür übliche Destillationskolonnen in Betracht, wie sie beispielsweise in: Kirk-Othmer, Encyclopedia of Chemical Technology, 3.Ed., Vol. 7, John Wiley & Sons, New York, 1979, Seite 870-881 beschrieben sind, wie Siebbodenkolonnen, Glockenbodenkolonnen, Packungskolonnen oder Füllkörperkolonnen.

Bevorzugt sind Destillationsapparturen, die vom Sumpf zum Kopf einen Druckabfall von 1 bis 1000 mbar, vorzugsweise 3 bis 300 mbar aufweisen, wobei vorteilhaft der Druck im Sumpf im Bereich von 10 bis 1000 mbar und im Kopf im Bereich von 30 bis 300 mbar liegen sollte.

Die Destillation kann man in mehreren, wie 2 oder 3 Kolonnen, vorteilhaft einer einzigen Kolonne durchführen.

Erfindungsgemäß weist das Destillationsgemisch auf mindestens einer, vorzugsweise 1 bis 15, besonders bevorzugt 1 bis 7, insbesondere 1, 2 oder 3 Ebenen der Destillationskolonne eine durchschnittliche mittlere Verweilzeit von mindestens 5 Minuten, vorzugsweise mindestens 15 Minuten, insbesondere mindestens 45 Minuten auf.

Vorzugsweise entnimmt man der Destillationskolonne das Destillationsgemisch auf mindestens einer Ebene, leitet es durch einen Verweilzeitbehälter und führt es der Destillationskolonne wieder zu. Die Rückführung kann dabei auf die Abzugsebene oder auf eine Ebene oberhalb oder unterhalb der Abzugsebene erfolgen.

Vorteilhaft kann der Rücklauf der Destillationskolonne zunächst durch einen Verweilzeitbehälter und anschließend in die Destillationskolonne zurückgeführt werden.

Das Entnehmen von Destillationsflüssigkeit aus der Kolonne, Leiten durch den Verweilzeitbehälter, Zurückführen in die Destillationskolonne und gegebenenfalls Umwälzung der Flüssigkeit im Verweilzeitbehälter kann mit an sich bekannten Apparaturen, wie Pumpen, erfolgen, wobei die Zurückführung auf die Abzugsebene der Destillationskolonne, insbesondere bei einer Bodenkolonne, oder auf eine oberhalb, insbesondere bei einer Packungskolonne, oder unterhalb der Abzugsebene liegende Ebene erfolgen kann.

Die Destillation der Mischung (I) kann vorteilhaft in Gegenwart von Kohlendioxid erfolgen.

Kohlendioxid kann dem Destillationsgemisch vor oder vorzugsweise während der Destillation in Form einer Verbindung, die unter den Destillationsbedingungen Kohlendioxid freisetzt, wie Ammoniumcarbonat, Ammoniumcarbamat oder Harnstoff oder Gemische solcher Verbindungen, wobei diese Verbindungen in reiner Form oder in einem flüssigen Verdünnungsmittel, wie einem oder mehreren Bestandteilen der Mischung (I), zugegeben werden können, oder in Form von festem, flüssigem oder vorzugsweise gasförmigem Kohlendioxid, beispielsweise in Form eines Kohlendioxid enthaltenden Gases oder insbesondere in Form von reinem, nur die üblichen Verunreinigungen enthaltendem gasförmigem Kohlendioxid, erfolgen.

Der Kohlendioxid-Gehalt im Destillationsgemisch sollte 0,1 bis 100 mol Kohlendioxid pro mol Iminfunktion des Imins (II) betragen.

Die Destillation der Mischung (I) kann vorteilhaft unter Zugabe einer gegenüber 6-Aminocapronsäurenitril unter den Destillationsbedingungen inerten Verbindung (III), deren Siedepunkt unter den Destillationsbedingungen über dem Siedepunkt von 6-Aminocapronsäurenitril liegt, erfolgen.

Als Verbindungen (III) kommen Verbindungen aus der Gruppe der Aromaten, der Aliphaten, wie acyclische und cyclische Aliphaten, und aliphatisch-aromatische Verbindungen in Betracht. Diese Verbindungen können Substituenten tragen, wie eine Hydroxyl-, Keto-, Ester-, Alkyl-, Aryl, Cycloalkyl-, Arylalkyl-Gruppe, vorzugsweise eine Nitril- oder Amino-Gruppe, oder mehrere gleiche oder unterschiedliche solcher Gruppen.

Die Verbindung (III) kann aus einer Verbindung oder Gemischen solcher Verbindungen bestehen.

Vorteilhaft sind solche Verbindungen (III), die sich auf einfache Weise, wie durch Hydrierung beispielsweise mit einem molekularen Wasserstoff enthaltenden Gas in Gegenwart eines Katalysators, zu Hexamethylendiamin, vorzugsweise 6-Aminocapronsäurenitril umsetzen lassen.

Die bei dieser Umsetzung erhaltenen Produkte können in das erfindungsgemäße Verfahren vorteilhaft erneut eingesetzt werden.

Die Differenz der Siedepunkte zwischen dem Amin der Mischung (I) und der Verbindung (III) sollte unter den Destillationsbedingungen 1 bis 200°C, vorzugsweise 5 bis 100°C betragen.

Als besonders vorteilhaft hat sich der Einsatz von Adipodinitril oder Mischungen enthaltend im wesentlichen Adipodinitril erwiesen.

Die Verbindung (III) kann zu der Mischung (I) vor oder während der Destillation zugegeben werden.

Die Zugabe der Verbindung (III) zu der Mischung (I) vor der Destillation kann in an sich bekannter Weise in üblichen Mischapparturen erfolgen.

Die Zugabe der Verbindung (III) zu der Mischung (I) während der Destillation kann durch Einspeisen der Verbindung (III) in die Destillationsapparatur vorzugsweise in den Sumpf-Bereich erfolgen.

Nach dem erfindungsgemäßen Verfahren wird das 6-Aminocapronsäurenitril als Kopfprodukt erhalten. Sofern die Mischung (I) Adipodinitril enthält, kann dieses insbesondere über einen Seitenabzug entnommen und vorteilhaft wieder der genannten vollständigen oder vorzugsweise partiellen Hydrierung zugeführt werden.

### Beispiele

In den Beispielen sind sämtliche Prozent-Angaben Gewichts-Prozente, soweit nichts anderes angegeben.

### Vergleichsbeispiel

275 g/h eines Gemisches enthaltend 0,061 % Tetrahydroazepin, 0,27 % Hexamethylendiamin, 48 % 6-Aminocapronsäurenitril, Rest Adipodinitril wurden einer Kolonne mit druckverlustarmer Gewebepackung entsprechend 32 theoretischen Trennstufen kontinuierlich zugeführt.

Am Kopf der Kolonne wurde ein Druck von 14 mbar eingestellt, konstant 125 g/h Destillat abgezogen und ein Rücklauf von 87 g/h aufgegeben.

Der über einen Bilanzzeitraum von 16 h im stationären Betrieb gesammelte Kopfaustrag enthielt neben 6-Aminocapronsäurenitril 0,6 % Hexamethylendiamin und 0,13 % Tetrahydroazepin.

### Beispiel 1

Es wurde wie im Vergleichsbeispiel verfahren, mit dem Unterschied, daß der Rücklauf vor der Zuführung auf die Kolonne über einen bei 100°C thermostatisierten Verweilzeitbehälter geleitet wurde. Die Verweilzeit in dem Behälter betrug 30 min.

Der über einen Bilanzzeitraum von 17 h im stationären Betrieb gesammelte Kopfaustrag enthielt neben 6-Aminocapronsäurenitril 0,6 % Hexamethylendiamin und 0,12 % Tetrahydroazepin.

### Beispiel 2

Es wurde wie im Vergleichsbeispiel verfahren, mit dem Unterschied, daß die obersten 3 theoretischen Trennstufen (Gewebepackung) gegen Glöckenböden mit einem Flüssigkeitsholdup von 45 ml/Boden ausgetauscht wurden, wobei sich auf diesen Böden eine Verweilzeit von insgesamt 30 Minuten ergab.

Der über einen Bilanzzeitraum von 17 h im stationären Betrieb gesammelte Kopfaustrag enthielt neben 6-Aminocapronsäurenitril 0,6 % Hexamethylendiamin und 0,11 % Tetrahydroazepin.

### Beispiel 3

Es wurde wie im Vergleichsbeispiel verfahren, mit dem Unterschied, daß die obersten 6 theoretischen Trennstufen (Gewebepackung) gegen Glöckenböden mit einem Flüssigkeitsholdup von 45 ml/Boden ausgetauscht wurden, wobei sich auf diesen Böden eine Verweilzeit von insgesamt 30 Minuten ergab.

Der über einen Bilanzzeitraum von 16 h im stationären Betrieb gesammelte Kopfaustrag enthielt neben 6-Aminocapronsäurenitril 0,6 % Hexamethylendiamin und 0,062 % Tetrahydroazepin.

### Beispiel 4

Es wurde wie im Vergleichsbeispiel verfahren, mit dem Unterschied, daß die obersten 10 theoretischen Trennstufen (Gewebepackung) gegen Glöckenböden mit einem Flüssigkeitsholdup von 45 ml/Boden ausgetauscht wurden, wobei sich auf diesen Böden eine Verweilzeit von insgesamt 30 Minuten ergab.

Der über einen Bilanzzeitraum von 16 h im stationären Betrieb gesammelte Kopfaustrag enthielt neben 6-Aminocapronsäurenitril 0,6 % Hexamethylendiamin und 0,013 % Tetrahydroazepin.

## Patentansprüche

1. Verfahren zur destillativen Abtrennung von 6-Aminocapronsäurenitril aus Mischungen (I), die 6-Aminocapronsäurenitril und ein Imin (II) enthalten, **dadurch gekennzeichnet, daß** man die Destillation in einer Destillationskolonne durchführt und daß dabei auf mindestens einer Ebene der Destillationskolonne das Destillationsgemisch eine durchschnittliche mittlere Verweilzeit von mindestens 5 Minuten aufweist.

2. Verfahren nach Anspruch 1, wobei das Destillationsgemisch von mindestens einer Ebene aus der Kolonne entnommen, durch einen Verweilzeitbehälter geleitet und anschließend wieder der Kolonne zugeführt wird.

3. Verfahren nach Anspruch 1, wobei der Rücklauf der Kolonne zunächst durch einen Verweilzeitbehälter und anschließend in die Destillationskolonne zurückgeführt wird.

4. Verfahren nach den Ansprüchen 1 bis 3, wobei man als Imin (II) ein zyklisches Imin einsetzt.

5. Verfahren nach den Ansprüchen 1 bis 4, wobei man als Imin (II) Tetrahydroazepin einsetzt.

6. Verfahren nach den Ansprüchen 1 bis 5, wobei man die Destillation im Bereich von 10 bis 1000 mbar durchführt.

## Claims

1. A process for distillative removal of 6-aminocapronitrile from mixtures (I) comprising 6-aminocapronitrile and an imine (II), which comprises performing the distillation in a distillation column using an average mean residence time for the distillation mixture of at least 5 minutes on at least one level of the distillation column.

2. A process as claimed in claim 1, wherein the distillation mixture is withdrawn from the column on at least one level, passed through a delay vessel and then returned into the column.

3. A process as claimed in claim 1, wherein the column reflux is first passed through a delay vessel before it is returned into the distillation column.

4. A process as claimed in any of claims 1 to 3, wherein a cyclic imine is used as imine (II).

5. A process as claimed in any of claims 1 to 4, wherein tetrahydroazepine is used as imine (II).

6. A process as claimed in any of claims 1 to 5, wherein the distillation is carried out within the range from 10 to 1000 mbar.

## Revendications

1. Procédé de séparation de nitrile d'acide 6-aminocaproïque à partir de mélanges (I) contenant du nitrile d'acide 6-aminocaproïque et une imine (II), **caractérisé en ce que** l'on entreprend la distillation dans une colonne de distillation et qu'au moins à un niveau de la colonne de distillation, le mélange de distillation présente un temps de séjour moyen d'au moins 5 minutes.

2. Procédé selon la revendication 1, où le mélange de distillation est soutiré d'au moins un niveau de la colonne, envoyé dans un réservoir de séjour et réinjecté ensuite dans la colonne.

3. Procédé selon la revendication 1,où le reflux de la colonne est envoyé d'abord dans un réservoir de séjour, puis renvoyé dans la colonne de distillation.

4. Procédé selon les revendications 1 à 3, où l'on met en oeuvre en tant qu'imine (II) une imine cyclique.

5. Procédé selon les revendications 1 à 4, où l'on met en oeuvre en tant qu'imine (II) de la tétrahydroazépine.

6. Procédé selon les revendications 1 à 5, où l'on entreprend la distillation dans la plage de 10 à 1000 mbar.
